# EUROPÄISCHE PATENTANMELDUNG

(11) **EP 1 875 942 A1**
(43) Veröffentlichungstag der Anmeldung: **09.01.2008**
(21) Anmeldenummer: 06115795.4
(22) Anmeldetag: 21.06.2006
(51) Int. Cl.: A61Q 13/00, A61K 8/27, A61K 8/19, A61K 8/34, A61K 8/31, A61K 8/37, A61K 8/33, A61K 8/35, A61K 8/49

(54) **Riechstoffhaltige Zusammensetzungen umfassend deliqueszente Stoffe**

(71) Anmelder: Symrise GmbH & Co. KG, 37603 Holzminden (DE)
(72) Erfinder: Eggers, Marcus, 37688, Beverungen (DE); Meier, Manfred, 37699, Fürstenberg (DE); Widder, Sabine, 37603, Holzminden (DE)
(74) Vertreter: Eisenführ, Speiser & Partner

(57) **Zusammenfassung**

Die vorliegende Erfindung betrifft eine Zusammensetzung umfassend oder bestehend aus:
A) 45 - 99 Gew.-% Ethanol, bezogen auf das Gesamtgewicht der Zusammensetzung,
B) einem oder mehreren Riechstoffen,
C) Wasser,
D) (i) einem oder mehreren deliqueszenten Stoffen.

## Beschreibung

Die vorliegende Erfindung betrifft bestimmte ethanolische Riechstoffzusammensetzungen (Riechstoffmischungen), die Verwendung deliqueszenter Stoffe zum Erreichen bestimmter Effekte in ethanolischen Riechstoffzusammensetzungen, Verfahren zur Herstellung erfindungsgemäßer ethanolischer Riechstoffzusammensetzungen sowie Verfahren zum Erzielen bestimmter Effekte in ethanol i-schen Riechstoffzusammensetzungen, wobei deliqueszente Stoffe eingesetzt werden, sowie Verfahren zur Vermittlung, Verstärkung oder Modifizierung eines Geruches auf der menschlichen Haut. Ein besonderer Aspekt der vorliegenden Erfindung betrifft die Verbesserung der Haftfestigkeit von ethanolischen Riechstoffzusammensetzungen auf Haut und/oder Haar.

Im Riechstoffbereich, insbesondere in der Feinparfümerie, ist es allgemein bekannt, dass es bei Anwendung ethanolischer Riechstoffzusammensetzungen (insbesondere Parfums, Eau de Toilette (EdT) etc.) auf der Haut durch die Verdampfung des Ethanols und/oder des regelmäßig vorhandenen Wassers zu einem Verlust insbesondere der leichtflüchtigen Kopfnote (Topnote) kommt, während die schwerer flüchtigen Noten eine hervorragende Haftung auf der Haut aufweisen und über einen langen Zeitraum von der Haut abgegeben und dementsprechend geruchlich wahrgenommen werden. Durch die schnellere Verdampfung der Kopfnote kommt es folglich im zeitlichen Verlauf zu einer deutlichen Veränderung des Geruchsprofils von auf Haut aufgetragenen ethanolischen Riechstoffzusammensetzungen. Ein ähnlicher Effekt tritt bei der Anwendung ethanolischer Riechstoffzusammensetzungen auf (menschlichem) Haar auf. Hierbei ist zu berücksichtigen, dass der beschriebene nachteilige Effekt insbesondere dann als problematisch empfunden wird, wenn eine ethanolische Riechstoffzusammensetzung (Riechstoffmischung) als leave-on-Produkt eingesetzt wird, das heißt auf der Haut und/oder dem Haar verbleiben soll, um von dort aus über einen längeren Zeitraum abgegeben zu werden. Weniger problematisch ist der Verlust insbesondere leichtflüchtiger Kopfnoten naturgemäß dort, wo eine (ethanolische) Riechstoffzusammensetzung lediglich eingesetzt wird, um ein kurzzeitigen geruchlichen Effekt zu bewirken.

Unter Haftung, Haftbarkeit und Substantivität wird im Rahmen des vorliegenden Textes die Haftung eines Riechstoffes auf Haut und Haar, insbesondere die Haftung auf menschlicher Haut (ausschließlich der (Mund)Schleimhaut) verstanden. Wenngleich also die vorliegende Erfindung hinsichtlich der Applikation ethanolischer Riechstoffzusammensetzungen auch die (Mund)Schleimhaut betrifft, ist doch festzustellen, dass die Bedeutung der vorliegenden Erfindung hinsichtlich der Aspekte Haut (ausschließlich (Mund)Schleimhaut) und/oder Haar (insbesondere menschliche Haut) von noch weit höherer Relevanz ist, denn bei Applikation einer ethanolischen Riechzusammensetzung auf die (Mund)Schleimhaut wird regelmäßig der negative Verdampfungseffekt überlagert und dominiert von Abwaschungseffekten (im Bereich der Mundschleimhaut beispielsweise unterstützt durch den Einfluss von Speichel).

In der parfümistischen Praxis wurden bereits zahlreiche Versuche unternommen, um die Haftbarkeit bzw. die Wahrnehmbarkeit insbesondere der Kopfnote ethanolischer Riechstoffzusammensetzungen insbesondere auf menschlicher Haut zu verlängern und somit eine gewisse geruchliche "Profilstabilität" zu erreichen.

So wird vielfach die Verwendung sogenannter Fixateure beschrieben, das heißt die Verwendung von Einzelstoffen oder Kombinationen von (Riech)Stoffen, die leichter flüchtigen Riechstoffen oder Riechstoffmischungen zugesetzt werden, um deren Verdampfungsgeschwindigkeit zu reduzieren (vgl. US 6,737,396).

In IP.COM #000033581D (veröffentlicht Januar 2005) wird die Verwendung von Hydroxyalkylharnstoff-Derivaten, insbesondere Hydroxyethylharnstoff, beschrieben, wodurch die Haftungseigenschaften von Parfüms in kosmetischen Anwendungen, insbesondere wässrig-ethanolischen Formulierungen, auf Haut und Haar verlängert wird.

In US 3,939,099 wird die Verwendung von Filmbildnern beschrieben, die sich in einem Wasser/Ethanolgemisch lösen und mit Riechstoff(mischungen) mischbar sind. Als Beispiele werden ionische und nichtionische Derivate von wasserlöslichen Polymeren genannt wie z.B. Polyvinylpyrrolidonderivate, quarternäre Polyvinylpyrrolidone mit Molgewichten im Bereich 50000 - 1000000, kationische Cellulosederivate und ähnliche. Während des Verdampfens von Ethanol kommt es zur Bildung eines Films auf der Haut, in den die Riechstoffe eingelagert sind.

US 6,210,688 beschreibt die Bildung und Verwendung eines geruchlosen Polymerfilms auf der Haut (basierend auf Vinylether-Copolymeren, Polyacrylaten, Methacrylaten, Polyestern, Polyfluorkohlenwasserstoffen, Polysaccariden), auf den anschließend ein Parfüm appliziert wird. Dadurch sollen "Reaktionen" mit der Haut unterbunden werden.

FR 2 747 306 beschreibt die Verwendung polymerer Kohlenwasserstoffe (Polyethylene mit Molekulargewichten zwischen 3000 und 30000). Da diese Polymere in Ethanol/Wasser unlöslich sind, ist ein entsprechendes Produkt wie z.B. Eau de Cologne, EdT oder ein Eau de Parfum trübe.

WO 2004/098556 beschreibt eine neuartige sprühbare und klare Parfümformulierung, die sich durch eine erhöhte Oberflächenspannung bzw. verringerte Berührungsfläche nach Applikation auszeichnet, die durch die Anwendung einer wirksamen Menge eines Polymers erreicht wird. Durch die kleinere Berührungsfläche wird das verbleibende Parfümöl nach dem Verdampfen des Ethanols auf einer kleineren Fläche konzentriert, von der die Riechstoffe langsamer abdampfen.

Filmbildner und/oder Polymere haben den Nachteil, dass sie nicht nur die Verdampfungsgeschwindigkeit der Kopfnote, sondern auch die aller anderen, schwerer flüchtigen Riechstoffe reduzieren, wodurch die Gesamtintensität merklich reduziert wird. Weiterhin können Filmbildner ein unangenehmes klebriges bzw. spannendes Hautgefühl hervorrufen.

EP 0 181 401 und EP 0 857 481 beschreiben gelartige Parfümzubereitungen, in denen die Diffusion und damit auch die Verdampfung insbesondere von leicht flüchtigen Riechstoffen reduziert wird. Die vorgeschlagene Gelbildung ist allerdings insbesondere für den Bereicht der Feinparfümerie (Feine Fragrance) nicht geeignet, da derartige Zubereitungen nicht sprühbar sind. Zudem wirkt sich die gelartige Formulierung nicht nur auf die Verdampfung der leichtflüchtigen Riechstoffe (Kopfnote) aus, sondern auch auf die Verdampfung von schwerer flüchtigen Basisnoten, was sich insgesamt in einer Reduktion der Geruchsintensität (Impact) bemerkbar macht.

Es war daher die primäre Aufgabe der vorliegenden Erfindung, eine (alternative) ethanolische Riechstoffzusammensetzung anzugeben, in der die eingesetzten Riechstoffe, insbesondere aber leichtflüchtige Riechstoffe und insbesondere Riechstoffe, welche die Kopfnote eines Duftes bilden, zu einer langen (gegenüber konventionellen Riechstoffzusammensetzungen verlängerte) Haftung insbesondere auf (menschlicher) Haut (damit ist Haut exklusive Schleimhaut gemeint) besitzen. Dabei sollten die vorstehend geschilderten Nachteile bisheriger Riechstoffzusammensetzungen möglichst vermieden werden.

Vorzugsweise sollte die anzugebende ethanolischer Riechstoffzusammensetzung einen oder mehrere Zusatzstoffe umfassen, welche die Haftung der (insbesondere leichtflüchtigen) Riechstoffe in der gewünschten Weise beeinflussen, aber ansonsten auf die sensorischen Eigenschaften der ethanolischen Riechstoffzusammensetzung keine oder nur eine äußerst geringe und daher tolerable Auswirkung haben.

Die anzugebenden ethanolischen Riechstoffzusammensetzungen sollten dabei vorzugsweise transparent (klar) und/oder sprühfähig sein.

Die primäre gestellte Aufgabe wird erfindungsgemäß gelöst duch eine Zusammensetzung umfassend oder bestehend aus:
A) 45 - 99 Gew.-% Ethanol, bezogen auf das Gesamtgewicht der Zusammensetzung,
B) einem oder mehreren Riechstoffen,
C) Wasser,
D) (i) einem oder mehreren deliqueszenten Stoffen. Vorzugsweise beträgt in einer erfindungsgemäßen Zusammensetzung der Anteil der Summe der Bestandteile A), B), C) und D) an der Zusammensetzung 95 Gew.-% oder mehr, bevorzugt 98 Gew.-% oder mehr.

Die erfindungsgemäßen Zusammensetzungen zeichnen sich insbesondere aus durch
- eine verlängerte Haftung auf Haut und/oder Haar, insbesondere auf menschlicher Haut (damit ist auch hier menschliche Haut gemeint, bei der es sich nicht um (Mund)Schleimhaut handelt), insbesondere der Topnote;
- einen über eine längere Zeit weitgehend gleichbleibenden Geruchseindruck nach Applikation auf menschliche Haut (siehe dazu die Beispiele unten)
- einen höheren Impact nach Applikation auf die menschliche Haut.

Im Rahmen des vorliegenden Textes wird unter einem "deliqueszenten Stoff' ein Stoff verstanden, der im reinen, festen Zustand an der Luft bei ausreichender Luftfeuchtigkeit verflüssigt.

Bevorzugt ist eine erfindungsgemäße Zusammensetzung, bei der im Bestandteil D)
(i) der eine oder die mehreren deliqueszenten Stoffe ein bzw. mehrere Salze sind.

Bevorzugt ist somit der Einsatz deliqueszenter Salze. Deliqueszente Salze verflüssigen sich im freien, festen Zustand an der Luft bei ausreichender Luftfeuchtigkeit durch Absorption von Luftfeuchtigkeit.

Vorzugsweise sind in einer erfindungsgemäßen Zusammensetzung die Anteile an Bestandteil A) (Ethanol) und Bestandteil D) so gewählt, dass Bestandteil D) in der Zusammensetzung vollständig und ohne Trübung gelöst vorliegt.

Bevorzugt sind erfindungsgemäße Zusammensetzungen, in denen Bestandteil D)
(i) ein oder mehrere deliqueszente Salze umfasst, die ausgewählt sind aus einer Gruppe G,
wobei die Gruppe G eine Gruppe ist bestehend aus: Ammoniumhydrogencarbonat, Ammoniumtricitrat, Ammoniumfluorid, Ammoniumformiat, Ammoniummagnesiumchlorid, Ammoniumpropionat, Ammoniumsulfamat, Ammoniumthiocyanat, Bariumiodid, Bariumbromid, Bariumchlorid, Bariumthiocyanat, Bariumnitrat, Cäsiumiodid, Cäsiumchlorid, Cäsiumhydroxid, Cäsiumcarbonat, Cäsiumfluorid, Calciumbromid, Calciumchlorid, Calciumiodid, Calciumnitrat, Calciumnitrit, Calciumthiocyanat, Cobaltdiacetat, Cobaltdibromid, Cobaltdichlorat, Cobaltdiperchlorat, Cobaltdiiodid, Kupferdibromid, Kupferdichlorat, Kupferdiperchlorat, Kupferfluorosilicat, Kupferdinitrat, Lithiumchlorat, Lithiumchlorid, Lithiumbromid, Lithiumsalicylat, Lithiumsulfid, Magnesiumacetat, Magnesiumbromid, Magnesiumchlorat, Magnesiumperchlorat, Magnesiumchlorid, Magnesiumiodid, Magnesiumnitrat, Mangandichlorid, Kaliumiodid, Kaliumformiat, Kaliumhydroxid, Silberperchlorat, Natriumperchlorat, Natriumhydroxid, Natriumphenolat, Natriumhypophosphit, Natriumhydrogensulfid, Natriumsulfid, Natriumthiocarbonat, Natriumthiocyanat, Titantetrabromid, Titantrichlorid, Zinkperchlorat, Zinkchlorid und Zinkthiocyanat.

Bevorzugte deliqueszente Salze (aus der Gruppe G) sind:
Ammoniumhydrogencarbonat, Ammoniumtricitrat, Ammoniumfluorid, Ammoniumformiat, Ammoniummagnesiumchlorid, Ammoniumpropionat, Ammoniumsulfamat, Ammoniumthiocyanat, Cäsiumiodid, Cäsiumchlorid, Cäsiumhydroxid, Cäsiumcarbonat, Cäsiumfluorid, Calciumbromid, Calciumchlorid, Calciumiodid, Calciumnitrat, Calciumthiocyanat, Kupferdinitrat, Lithiumchlorat, Lithiumchlorid, Lithiumbromid, Lithiumsalicylat, Lithiumsulfid, Magnesiumacetat, Magnesiumbromid, Magnesiumchlorat, Magnesiumperchlorat, Magnesiumchlorid, Magnesiumiodid, Magnesiumnitrat, Mangandichlorid, Kaliumiodid, Kaliumformiat, Natriumperchlorat, Natriumphenolat, Natriumhypophosphit, Natriumthiocyanat, Zinkperchlorat, Zinkchlorid und Zinkthiocyanat.

Besonders bevorzugte deliqueszente Salze (aus der Gruppe G) sind:
Ammoniumhydrogencarbonat, Ammoniumfluorid, Ammoniummagnesiumchlorid, Ammoniumpropionat, Ammoniumthiocyanat, Cäsiumchlorid, Cäsiumfluorid, Calciumbromid, Calciumchlorid, Calciumiodid, Calciumnitrat, Kupferdinitrat, Lithiumchlorid, Lithiumbromid, Magnesiumacetat, Magnesiumbromid, Magnesiumchlorid, Magnesiumiodid, Magnesiumnitrat, Kaliumiodid und Zinkchlorid.

Am meisten bevorzugte deliqueszente Salze (aus der Gruppe G) sind:
Calciumchlorid, Calciumnitrat, Kupferdinitrat, Lithiumchlorid und Magnesiumchlorid, insbesondere Calciumchlorid und Calciumnitrat.

In einem erfindungsgemäßen Verfahren zur Herstellung einer erfindungsgemäßen Zusammensetzung (insbesondere in einer der vorstehend als bevorzugt angegebenen Ausgestaltungen) werden
A) 45 - 99 Gew.-% Ethanol,
B) ein oder mehr Riechstoffe (insbesondere leichtflüchtige Riechstoffe),
C) Wasser,
sowie gegebenenfalls weitere Bestandteile mit
D) (i) einem oder mehreren deliqueszenten Stoffen und/oder (ii) zwei oder mehr Verbindungen, die in der resultierenden Zusammensetzung zusammen in-situ einen deliqueszenten Stoff bilden, vermischt. Die Gewichtsprozentangabe ist hierbei wiederum bezogen auf das Gesamtgewicht der resultierenden Zusammensetzung nach dem Mischen.

Die "zwei oder mehr Verbindungen, die in der resultierenden Zusammensetzung zusammen in-situ einen deliqueszenten Stoff bilden" sind im Rahmen des vorliegenden Textes solche, die gemeinsam bei bzw. nach Einarbeitung in eine erfindungsgemäße Zusammensetzung ein deliqueszentes Salz bilden.

Gemäß Alternative (ii) können also beispielsweise Verbindungen eingesetzt werden, die in-situ einen deliqueszenten Stoff bilden, also zum Beispiel Ammoniumchlorid und Calciumpropionat, die nach dem Vermischen mit dem Bestandteilen A), B) und C) (sowie gegebenenfalls weiteren Bestandteilen, siehe unten, insbesondere Bestandteile E) und F)) in Lösung vorliegen, wobei die Lösung dann als formal Calciumchlorid sowie Ammoniumpropionat umfassend aufgefasst werden kann.

Vorzugsweise liegt Bestandteil D) in einer erfindungsgemäßen Zusammensetzung vollständig gelöst vor, insbesondere dann, wenn ein deliqueszentes Salz eingesetzt oder in-situ gebildet wird.

Nach eigenen Erkenntnissen hängt die Wirksamkeit des Bestandteils D) einer erfindungsgemäßen Zusammensetzung (also insbesondere die Wirksamkeit eines deliqueszenten Salzes) davon ab, wie deliqueszent der bzw. die eingesetzten deliqueszenten Stoffe im reinen, festen Zustand sind. Je stärker die Deliqueszenz des Bestandteils D) ausgeprägt ist, desto stärker ausgeprägt sind gemäß eigenen Untersuchungen die beschriebenen Wirkungen in einer erfindungsgemäßen ethanolischen Riechstoffzusammensetzung. Insbesondere wurde mit zunehmender Deliqueszenz eine zunehmend verbesserte Haftung (insbesondere der Kopfnote) auf Haut und/oder Haar beobachtet, insbesondere auf menschlicher Haut.

Ferner wurde ein proportionaler Zusammenhang zwischen der Menge der eingesetzten deliqueszenten Stoffe (insbesondere deliqueszenten Salze) und der Stärke der beschriebenen Wirkungen beobachtet. Insbesondere wurde bei Verdoppelung der Menge an zugesetztem deliqueszentem Salz auch in etwa eine Verdoppelung der Wirkung in einer erfindungsgemäßen Zusammensetzung beobachtet, insbesondere eine verbesserte Haftung (insbesondere der Kopfnote) auf Haut und/oder Haar, insbesondere auf menschlicher Haut (d. h. nicht (Mund)Schleimhaut).

In einer erfindungsgemäßen Zusammensetzung (insbesondere in einer der vorstehend als bevorzugt angegebenen Ausgestaltungen) liegt Bestandteil D) vorzugsweise in einer wirksamen Menge vor, das heißt in einer Menge, bei der in der Zusammensetzung durch den Bestandteil D) einer oder mehrere Effekte bewirkt werden, die ausgewählt sind aus der Gruppe von Effekten bestehend aus:
- Reduzierung der Verdampfung von Riechstoffen, insbesondere unmittelbar nach der Applikation der erfindungsgemäßen Zusammensetzung und insbesondere von leichtflüchtigen Riechstoffen (insbesondere Kopfnote); dies gilt insbesondere für (leichtflüchtige) Riechstoffe, die üblicherweise durch die Verdampfung von Ethanol/Wasser kurz nach der Applikation einer wässrig-ethanolhaltigen Zusammensetzung mitgerissen werden. Mit diesem Effekt ist keine nennenswerte Reduzierung der Verdampfung schwerer flüchtiger Riechstoffe verbunden; dies gilt insbesondere für Riechstoffe die auch bei Abwesenheit eines Bestandteiles D) in einer wässrig-ethanolhaltigen Zusammensetzungen eine teiles D) in einer wässrig-ethanolhaltigen Zusammensetzungen eine mehr oder weniger gleichbleibende Verdampfung aufweisen.
- zeitliche Stabilisierung des Geruchsprofils, das heißt, dass über einen längeren Zeitraum ein zumindest im Wesentlichen gleichbleibendes Geruchsprofil erreicht wird;
- Erhöhung oder zeitliche Stabilisierung des geruchlichen Impact (der wahrgenommenen Geruchsstärke); das heißt, dass der geruchliche Impact (die wahrgenommene Geruchsstärke) über einen langen Zeitraum nicht oder nicht nennenswert verringert wird; abhängig von den jeweiligen eingesetzten Konzentrationen der Bestandteil D) innerhalb der erfindungsgemäßen Zusammensetzung wird der Impact über einen mehr oder weniger langen Zeitraum erhöht;
- Verlängerung der Haftung der Zusammensetzung, insbesondere der leichtflüchtigen Riechstoffe (Kopfnote), auf Haut und/oder Haar, insbesondere auf menschlicher Haut;
- Erhöhung oder Verlängerung der Diffusivität (Raumwirkung) der Zusammensetzung, insbesondere der leichtflüchtigen Riechstoffe (Kopfnote), auf Haut und/oder Haar, insbesondere auf menschlicher Haut;
- Vermittlung eines angenehmen Hautgefühls.

Eine erfindungsgemäße Zusammensetzung hinterlässt vorzugsweise kein Film auf Haut und/oder Haar und wird somit nicht als störend empfunden. Kurze Zeit nach dem Auftragen (das heißt nach dem Trocknen) ist eine erfindungsgemäße Zusammensetzung regelmäßig nicht mehr sichtbar (Ausnahme: Haarpflegemittel wie beispielsweise Haarspray, bei denen die Filmbildung gewünscht ist).

Erfindungsgemäße Zusammensetzungen sind demnach - kurz zusammengefasst - Zusammensetzungen mit einer besonders guten Haftung der Kopfnote einer Riechstoffmischung sowie einem verlängerten und/oder verstärkten Impact der Kopfnote, wobei eine Minderung der Herz- oder Basisnote nicht oder zumindest nicht in nennenswerten Umfang zu beobachten ist.

Bevorzugt ist eine erfindungemäße Zusammensetzung, vorzugsweise in einer der vorstehend als bevorzugt angegebenen Ausgestaltungen, umfassend:
A) 50 - 98,5 Gew.-%, vorzugsweise 60 - 95 Gew.-%, Ethanol,
   und/oder
B) 0,1 - 35 Gew.-%, vorzugsweise 0,5 - 25 Gew.-%, einer Riechstoffmischung umfassend 2, 3, 4, 5, 6, 7, 8, 9, 10 oder mehr Riechstoffe,
   und/oder
C) 0,5 - 40 Gew.-%, vorzugsweise 0,5 - 25 Gew.-%, Wasser,
   und/oder
D) 0,5 - 20 Gew.-%, vorzugsweise 2-12 Gew.-%, bevorzugt 3 - 8 Gew.-%, eines oder mehrerer deliqueszenter Salze.

Die Gewichtsprozentangaben sind jeweils bezogen auf das Gesamtgewicht der Zusammensetzung. Vorzugsweise sind die zu den Bestandteilen A), B), C) und D) genannten bevorzugten Gewichtsanteile gleichzeitig eingestellt.

Als zusätzlichen Bestandteil kann eine erfindungsgemäße Zusammensetzung (insbesondere in einer der vorstehend als bevorzugt gekennzeichneten Ausgestaltungen) umfassen:
E) gegebenenfalls Glycerin, Ethylenglykol, 1,2-Propylenglykol, Diethylphthalat und/oder ein geradkettiges Alkandiol mit 6 bis 12 C-Atomen.

Eine besonders bevorzugte erfindungsgemäße Zusammensetzung umfasst oder besteht aus:
A) 60 - 95 Gew.-% Ethanol,
B) 1 - 25 Gew.-% einer Riechstoffmischung umfassend 2, 3, 4, 5, 6, 7, 8, 9, 10 oder mehr Riechstoffe,
C) 1 - 22 Gew.-% Wasser,
D) 2 - 12 Gew.-%, bevorzugt 3 - 8 Gew.-%, eines oder mehrerer deliqueszenter Salze (vorzugsweise eines oder mehrere der vorstehend als bevorzugt gekennzeichneten Salze), sowie
E) gegebenenfalls Glycerin, Ethylenglykol, 1,2-Propylenglykol, Diethylphthalat und/oder einem geradkettigen Alkandiol mit 6 bis 12 C-Atomen. Die Gewichtsprozentangaben sind jeweils bezogen auf das Gesamtgewicht der Zusammensetzung.

Der oder die optionalen Bestandteile E) Glycerin, Ethylenglykol, 1,2-Propylenglykol, Diethylphthalat und/oder geradkettiges Alkandiol mit 6 bis 12 C-Atomen (insbesondere 1,2-Pentandiol, 1,2-Hexandiol und 1,2-Octandiol und deren Mischungen) können, insbesondere in einem Rasierwasser als Beispiel einer erfindungsgemäßer Zusammensetzung, bis zu 6 Gew.-% enthalten sein. Der oder die optionalen Bestandteile E) Glycerin, Ethylenglykol, 1,2-Propylenglykol, 1,2-Pentandiol, 1,2-Hexandiol und/oder 1,2-Octandiol können als Lösungsvermittler fungieren, so dass eventuelle unerwünschte Trübungen und/oder Entmischungen unterbleiben.

Vorzugsweise besitzt eine erfindungsgemäße Zusammensetzung eine, zwei oder sämtliche Eigenschaften aus der Gruppe bestehend aus: transparent (klar), echte Lösung, sprühfähig (sprühbar). Vorzugsweise ist eine erfindungsgemäße Zusammensetzung also transparent (klar) und/oder eine echte Lösung und/oder sprühfähig (sprühbar).

Vorzugsweise umfasst Bestandteil B) einer erfindungsgemäßen Zusammensetzung (insbesondere in einer der vorstehend als bevorzugt gekennzeichneten Ausgestaltungen) einen oder mehrere Riechstoffe aus der Gruppe bestehend aus:
alpha-Pinen, beta-Pinen, Limonen, 6-Methyl-5-hepten-2-on, Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al, 3-Hexenylmethylcarbonat, Benzaldehyd, Linalool, Linalylacetat, Caryophyllen, Ethyllinalool, Citral, Neral, Geranial, Benzylalkohol, p-Anisaldehyd, Menthol, Hexylacetat, Citronellol, Nerol, Geraniol und 2-Phenylethylalkohol. Bei Einsatz der besagten Riechstoffe sind die vorstehend genannten Effekte besonders ausgeprägt.

Die genannten bevorzugten Riechstoffe des Bestandteils B) können dabei, sofern zutreffend, in Form ihrer jeweiligen Diastereomere, Enantiomere und/oder Doppelbindungsisomere vorliegen. So können diese als (E)/(Z)-Isomere, als beliebiges Gemisch der Enantiomeren, insbesondere als Racemat, oder auch als beliebiges Gemisch der entsprechenden Diastereomeren, vorliegen.

Eine bevorzugte erfindungsgemäße Zusammensetzung umfasst als Bestandteil B) eine Riechstoffmischung mit 2, 3, 4, 5, 6, 7, 8, 9, 10 oder mehr Riechstoffen, umfassend oder bestehend aus
i) einer Kopfnote, enthaltend oder bestehend aus einem oder mehreren Riechstoffen mit einem jeweiligen Dampfdruck bei 25°C von größer oder gleich 1,333 Pa.

Vorzugsweise umfasst der Bestandteil B) einer erfindungsgemäßen Zusammensetzung also zumindest einen Riechstoff, der besonders leichtflüchtig ist (Dampfdruck bei 25 °C von größer oder gleich 1,333 Pa) und die Kopfnote bildet oder Bestandteile der Kopfnote ist. Generell wird im Rahmen des vorliegenden Textes unter einem leichtflüchtigen Riechstoff ein sensorisch wirksamer Stoff mit einem Dampfdruck von größer oder gleich 1,333 Pa (entsprechend etwa 0,01 Torr) bei 25°C verstanden, wobei allerdings die Verwendung von leichtflüchtigen Riechstoffen mit einem Dampfdruck von größer oder gleich 2,000 Pa (entsprechend etwa 0,015 Torr), insbesondere die Verwendung von Riechstoffen mit einem Dampfdruck von größer oder gleich 2,666 Pa (entsprechend etwa 0,02 Torr), jeweils bei 25°C, als Kopfnote oder Bestandteil der Kopfnote häufig vorteilhaft ist.

Bei Einsatz der im Folgenden genannten Riechstoffe oder deren Mischungen waren die oben genannten Effekte besonders ausgeprägt, weshalb diese Riechstoffe im Sinne der vorliegenden Erfindung am meisten bevorzugt sind:
alpha-Pinen, D-Limonen, 6-Methyl-5-hepten-2-on, Octanal, Eucalyptol (1,8-Cineol), cis-Rosenoxid, trans-Rosenoxid, Dihydromyrcenol (2,6-Dimethyl-7-octen-2-ol), d-Menthon, I-Menthon, 2,6-Dimethyl-5-hepten-1-al, (Z)-3-Hexenylmethylcarbonat, Linalool, Linalylacetat, Citral, Neral, Geranial, I-Menthol, racemisches Menthol, Hexylacetat, Citronellol und 2-Phenylethylalkohol.

Bevorzugt ist eine erfindungsgemäße Zusammensetzung, in der Bestandteil B) eine Riechstoffmischung mit 2, 3, 4, 5, 6, 7, 8, 9, 10 oder mehr Riechstoffen ist, umfassend oder bestehend aus:
i) einer Kopfnote, enthaltend oder bestehend aus einem oder mehreren Riechstoffen mit einem jeweiligen Dampfdruck bei 25°C von größer oder gleich 1,333 Pa, und
ii) einem oder mehreren Riechstoffen mit einem jeweiligen Dampfdruck bei 25°C von kleiner als 1,333 Pa.

Neben einer Kopfnote sind also in einer besonders bevorzugten erfindungsgemäßen Zusammensetzungen auch schwerer flüchtige Riechstoffe vorhanden.

Bevorzugt ist eine erfindungsgemäße Zusammensetzung, in der Bestandteil B) eine Riechstoffmischung mit 2, 3, 4, 5, 6, 7, 8, 9, 10 oder mehr Riechstoffen ist, umfassend oder bestehend aus:
i) einer Kopfnote, enthaltend oder bestehend aus 2, 3, 4, 5, 6 oder mehr Riechstoffen mit einem jeweiligen Dampfdruck bei 25°C von größer oder gleich 1,333 Pa, und
ii) 2, 3, 4, 5, 6 oder mehr Riechstoffen mit einem jeweiligen Dampfdruck bei 25°C von kleiner als 1,333 Pa.

Eine erfindungsgemäße Zusammensetzung kann neben den Bestandteilen A), B), C), D) sowie gegebenenfalls E) als zusätzlichen Bestandteil, das heißt als Bestandteil der sich nicht unter die obigen Definitionen zu den Bestandteilen A), B), C), D) und E) subsumieren lässt, zusätzlich eine oder mehrere Substanzen umfassen aus der Gruppe bestehend aus:
F) Antioxidantien (wie beispielweise BHT (p-tert.-Butylhydroxytoluol), BHA (p-tert.-Butylhydroxyanisol), Tocopherol (Vitamin E), Tocopherylacetat), hautpflegende Stoffe (alpha-Bisabolol, Allantoin, Aloe vera, Panthenol, Kamillenextrakt), Citronensäure, Vitamin C, UV-Filter, Emulgatoren, Fettalkohole, C₈-C₃₀-Fettsäurealkylester, Farbstoffe.

Bestandteil F) liegt in einer bevorzugten erfindungsgemäßen Zusammensetzung vorzugsweise lediglich in einer Menge von 2 Gew.-% oder weniger vor, bezogen auf das Gesamtgewicht der Zusammensetzung.

Die erfindungsgemäßen Zusammensetzungen sind in vielen parfümierten, insbesondere kosmetischen Produkten einsetzbar.

Es sei angemerkt, dass einige der in erfindungsgemäßen Zusammensetzungen bevorzugt verwendeten Riechstoffe als sensorisch wirksame Stoffe in üblichen alkoholischen Mundpflegeprodukten (mundhygienischen Produkten) eingesetzt werden, zum Beispiel Menthol, Menthon, Eucalyptol, Linalool, alpha-Pinen. Da es jedoch die primäre Aufgabe der vorliegenden Erfindung war, die Haftung der Kopfnote einer Riechstoffmischung auf Haut und/oder Haar zu verbessern, und zwar insbesondere unter leave-on-Bedingungen, bei denen die Riechstoffzusammensetzung nicht von Haut und/oder Haar abgewaschen wird, versteht es sich, dass es sich bei einer erfindungsgemäßen Zusammensetzung vorzugsweise nicht um ein Mundpflegeprodukt (mundhygienisches Produkt) handelt. So werden in üblichen Mundhygieneprodukten beispielsweise häufig folgende Salze eingesetzt, die in erfindungsgemäßen Zusammensetzungen vorzugsweise nicht oder in Konzentrationen vorhanden sind, die in Mundhygieneprodukten unüblich sind:
Natriummonofluorphosphat, Zinndifluorid, Zinkcitrat, Zinksulfat, Zinnpyrophosphat, Zinndichlorid, Kaliumcitrat, Kaliumnitrat, Kaliumchlorid, Strontiumchlorid, Cu-Salze, Alkalifluoride wie Na-fluorid, Erdalkalifluoride, Ammoniumfluorid.

Besonders bevorzugt sind erfindungsgemäße Zusammensetzungen sogar gänzlich frei von Zinnsalzen und/oder Zinksalzen und/oder Kupfersalzen (mit Ausnahme von Kupferdinitrat) und/oder Alkalifluoriden und/oder Erdalkalifluoriden. Besonders bevorzugt ist keines der genannten Salze in einer erfindungsgemäßen Zusammensetzung anwesend.

Zudem besitzen mundhygienische Zusammensetzungen häufig aufgrund der Anwesenheit von Aromastoffen wie Menthol, Menthon und Eucalyptol einen Pfefferminz-, Krauseminz- (Spearmint) oder Eukalyptusgeruch und/oder -geschmack. Bevorzugte erfindungsgemäße Zusammensetzungen weisen einen solchen Geruch und/oder Geschmack nicht auf.

Vorzugsweise sind erfindungsgemäße Zusammensetzungen frei von Pfefferminzöl, Krauseminzöl und/oder Eukalyptusöl.

Vorzugsweise ist eine erfindungsgemäße Zusammensetzung ausgewählt aus der Gruppe bestehend aus (der bevorzugte maximale Ethanolgehalt ist jeweils in Klammern angegeben): Extrait, Eau de Parfum, Eau de Toilette, Eau de Cologne (jeweils bis zu 99 Gew.-%), Splash Cologne, Rasierwasser, Deo-Formulierungen, Haarfestiger, Haarspray, Haargele (jeweils bis zu 50 Gew.-%), Gesichtswasser (bis zu 70 Gew.-%), Haarwasser (bis zu 75 Gew.-%), Handdesinfektionsmittel (bis zu 99 Gew.-%), Flüssigkeiten und Emulsionen für Erfrischungstücher (bis zu 90 Gew.-%).

Vorzugsweise handelt es sich bei den erfindungsgemäßen Zusammensetzungen um sogenannte "leave-on" - Produkte, d.h. um Produkte, die auf Haut und/oder Haar verbleiben und in der Regel nicht abgewaschen werden. Hierzu zählen insbesondere Eau de Parfum, Eau de Toilette, Eau de Cologne und Rasierwasser.

Daneben können erfindungsgemäße Zusammensetzungen z. B. auch in einer der folgenden (allerdings weniger bevorzugten) Formen vorliegen: Deospray, Deo-Roller (Roll-on), Haarspray. Hierbei handelt es sich ebenfalls um "leave-on" - Produkte.

Die vorliegende Erfindung betrifft auch die Verwendung eines deliqueszenten Stoffes (wie oben definiert, vorzugsweise eines oben als besonders bevorzugt angegebenen deliqueszenten Stoffes) als Mittel zur
- Reduzierung der Verdampfung von Riechstoffen in einer ethanolischen Riechstoffzusammensetzung, insbesondere unmittelbar nach der Applikation und insbesondere von leichtflüchtigen Riechstoffen;
- zeitlichen Stabilisierung des Geruchsprofils einer ethanolischen Riechstoffzusammensetzung;
- Erhöhung oder zeitlichen Stabilisierung des geruchlichen Impact (der wahrgenommenen Geruchsstärke) einer ethanolischen Riechstoffzusammensetzung;
- Verlängerung der Haftung von Riechstoffen einer ethanolischen Riechstoffzusammensetzung, insbesondere der leichtflüchtigen Riechstoffe (Kopfnote), auf Haut und/oder Haar, insbesondere auf menschlicher Haut;
- Erhöhung oder Verlängerung der Diffusivität (Raumwirkung) einer ethanolischen Riechstoffzusammensetzung, insbesondere der leichtflüchtigen Riechstoffe (Kopfnote), auf Haut und/oder Haar, insbesondere auf menschlicher Haut; und/oder
- Vermittlung oder Verstärkung eines angenehmen Hautgefühls einer ethanolischen Riechstoffzusammensetzung.

Die vorliegende Erfindung betrifft ferner ein Verfahren zur
- Reduzierung der Verdampfung von Riechstoffen in einer ethanolischen Riechstoffzusammensetzung, insbesondere unmittelbar nach der Applikation und insbesondere von leichtflüchtigen Riechstoffen,
- zeitlichen Stabilisierung des Geruchsprofils einer ethanolischen Riechstoffzusammensetzung,
- Erhöhung oder zeitlichen Stabilisierung des geruchlichen Impact (der wahrgenommenen Geruchsstärke) einer ethanolischen Riechstoffzusammensetzung,
- Verlängerung der Haftung von Riechstoffen einer ethanolischen Riechstoffzusammensetzung, insbesondere der leichtflüchtigen Riechstoffe (Kopfnote), auf Haut und/oder Haar, insbesondere auf menschlicher Haut,
- Erhöhung oder Verlängerung der Diffusivität (Raumwirkung) einer ethanolischen Riechstoffzusammensetzung, insbesondere der leichtflüchtigen Riechstoffe (Kopfnote), auf Haut und/oder Haar, insbesondere auf menschlicher Haut und/oder
- Vermittlung oder Verstärkung eines angenehmen Hautgefühls einer ethanolischen Riechstoffzusammensetzung,
mit folgendem Schritt:
Mischen von
   A) 45 - 99 Gew.-% Ethanol,
   B) einem oder mehreren Riechstoffen,
   C) Wasser, sowie gegebenenfalls weiteren Bestandteilen mit
   D) (i) einem oder mehreren deliqueszenten Stoffen und/oder (ii) zwei oder mehr Verbindungen, die in der resultierenden Zusammensetzung zusammen in-situ einen deliqueszenten Stoff bilden,
wobei die Gewichtsprozentangabe bezogen ist auf das Gesamtgewicht der resultierenden Zusammensetzung nach dem Mischen.

Die erfindungsgemäßen Zusammensetzungen lassen sich auf einfache Weise durch Mischen der Einzelkomponenten der Bestandteile A), B), C) und D) sowie gegebenenfalls E) und F) oder der Bestandteile A) bis D) sowie gegebenenfalls E) und F) oder der gegebenenfalls vorgemischten Bestandteile A), B), C) und D) sowie gegebenenfalls E) und F) herstellen. Dabei ist die Reihenfolge der Kontaktierung der Einzelkomponenten beziehungsweise Bestandteile nicht kritisch und kann variiert werden.

Die vorliegende Erfindung betrifft auch ein Verfahren zur Vermittlung, Verstärkung oder Modifizierung eines Geruches auf der menschlichen Haut, mit folgendem Schritt:
- Applizieren einer erfindungsgemäßen Zusammensetzung (bevorzugt einer Zusammensetzung, die oben als bevorzugt angegeben ist) auf die menschliche Haut.

Die Teile und Prozentangaben der nachfolgenden Beispiele beziehen sich jeweils auf das Gewicht.

### Beispiele

### Beispiel 1: Erfindunqsqemäß einzusetzende Kopfnote als zu verwendender Bestandteil B)

| **Nr.** | **Komponente** | **Dampfdruck (torr) bei 25 °C** | **Gew.-%** |
|---|---|---|---|
| 1 | Limonen | 2,330 | 10 |
| 2 | Hexylacetat | 1,560 | 5 |
| 3 | Octanal | 1,170 | 5 |
| 4 | Methylheptenon | 2,700 | 5 |
| 5 | 2,6-Dimethyl-5-hepten-1-al | 0,759 | 5 |
| 6 | Rosenoxid cis | 0,660 | 5 |
| 7 | Rosenoxid trans | 0,660 | 5 |
| 8 | Cis-3-Hexenol | 0,554 | 5 |
| 9 | Dihydromyrcenol | 0,124 | 5 |
| 10 | Menthon | 0,278 | 5 |
| 11 | (Z)-3-Hexenylmethylcarbonat | 0,187 | 5 |
| 12 | Linalool | 0,0577 | 10 |
| 13 | Linalylacetat | 0,0826 | 5 |
| 14 | Menthol | 0,0232 | 5 |
| 15 | Citronellol | 0,013 | 8 |
| 16 | Citral | 0,149 | 2 |
| 17 | Phenylethylalkohol | 0,0161 | 5 |
| 18 | Diethylphthalat (Lösungsmittel) | | 2,5 |
| 19 | Propylenglykol (Lösungsmittel) | | 2,5 |
| | Total: | | 100,0 |

### Beispiel 2: Erfindungsgemäß einzusetzende Aromastoffkomposition (synthetisches Pfefferminzöl) als zu verwendender Bestandteil B)

| | **Aroma A1** Gewichtsteile |
|---|---|
| Isobutyraldehyd | 0,5 |
| 3-Octanol | 0,5 |
| Dimethylsulfid | 0,5 |
| Trans-2-Hexenal | 1,0 |
| Cis-3-Hexenol | 1,0 |
| 4-Terpineol, natürlich | 1,0 |
| Isopulegol | 1,0 |
| Piperiton, natürlich, aus Eucalyptus | 2,0 |
| Linalool | 3,0 |
| 8-Ocimenylactetat, 10% in Triacetin | 5,0 |
| Isoamylalkohol | 10,0 |
| Isovaleraldehyd | 10,0 |
| Alpha-Pinen | 25,0 |
| Beta-Pinen, natürlich | 25,0 |
| Neomenthol, racemisch | 40,0 |
| Eucalyptol (1,8-Cineol), natürlich | 50,0 |
| L-Menthylacetat | 70,0 |
| L-Menthon | 220,0 |
| D-Isomenthon | 50,0 |
| L-Menthol | 484,5 |
| Total: | 1000,0 |

### Beispiel 3:

### Headspace Analyse von Haut - Verdampfungsrate einzelner Riechstoffe

**Test-Zusammensetzungen**

| **Inhaltsstoffe** | **P1** **Gew.-%** | **Ref1** **Gew.-%** |
|---|---|---|
| CaCl₂ | 5 | - |
| Zu untersuchender Einzelriechstoff (siehe unten) | 0,125 | 0,125 |
| Ethanol | 75 | 78,9 |
| Wasser | 19,875 | 20,975 |
| Total: | 100 | 100 |

Mittels einer Mikroliterspritze wurden 50 µl der jeweiligen Parfümformulierung P1 bzw. Ref1 (nicht erfindungsgemäß) auf eine definierte Fläche (2,5 x 2,5cm) des Unterarms der Probanden aufgetragen.

5 Minuten nach der Applikation wurde die Verdampfungsrate der einzelnen Riechtsoffe mittels dynamischer Headspace - Methodik gemessen. Hierbei wurde ein definierter Luftstrom durch eine auf den Unterarm angebrachte Glasglocke geleitet und die Riechstoffe an aktivkohlehaltigen Probenröhrchen mittels Adsorption angereichert. Die Anreicherung erfolgte während eines Zeitraumes von insgesamt 5 Stunden. Die Probenröhrchen wurden jeweils nach 10, 15, 25, 40 Minuten, 1 Stunde, 2, 3, 4, und 5 Stunden gewechselt und einzeln analysiert.

Die angereicherten Riechstoffe werden mit Diethylether eluiert und nach Zugabe eines Standards mittels GC/MS quantifiziert.

In den Figuren 1 bis 8 ist jeweils die absolute Menge des jeweils untersuchten Riechstoffes (in µg je Liter Luft, y-Achse) in Abhängigkeit von der Zeit (in Stunden, x-Achse) graphisch dargestellt. Es ist bei diesen Figuren zu beachten, dass die y-Achse eine logarithmische Skala aufweist. Der Ergebnisse der jeweiligen erfindungsgemäßen Zusammensetzung sind mit einem Kreis markiert, die der jeweiligen Referenzproben mit einem Dreieck.

Die Figuren 1 bis 5 zeigen exemplarisch den Verdampfungsverlauf für die erfindungsgemäß bevorzugt einzusetzenden leichtflüchtigen Riechstoffe Rosenoxid (Figur 1), Eucalyptol (Figur 2), Menthon (Figur 3), Linalool (Figur 4) und Dihydromyrcenol (Figur 5) nach Anwendung der erfindungsgemäßen Zusammensetzung (P1) sowie der Referenzprobe (Ref1).

Die Figuren 6 bis 8 zeigen exemplarisch den Verdampfungsverlauf für die schwerer flüchtigen, d.h. nicht leichtflüchtigen, Riechstoffe Synambran (3a,6,6,9a-Tetramethyldodecahydronaphtho[2,1-b]furan) (Figur 6), 8-Cyclohexdecenon (Figur 7) und Cedrol (Figur 8).

Die Ergebnisse zeigen für erfindungsgemäß bevorzugte Zusammensetzungen (Figuren 1 bis 5) für leichtflüchtige Riechstoffe (Kopfnote) über den gesamten Zeitraum deutlich höhere Headspace-Konzentrationen, während die schwerer flüchtigen Riechstoffe (Figuren 6 bis 8), deren Verdampfungsrate über den gesamten Applikationscyclus nahezu konstant ist, weitgehend unbeeinflusst bleiben.

In erfindungsgemäßen Zusammensetzungen ist somit insbesondere die Wahrnehmbarkeit von leichtflüchtigen Riechstoffen verlängert und verstärkt.

### Beispiel 4: Eau de Cologne

| **Inhaltsstoffe** | **P2** **Gew.-%** | **Ref2** **Gew.-%** |
|---|---|---|
| CaCl2 | 5 | - |
| Riechstoffmischung (Kopfnote) aus Beispiel 1 | 5 | 5 |
| Ethanol | 72 | 76 |
| Wasser | 18 | 19 |
| Total: | 100 | 100 |

Die Zusammensetzung Ref2 ist ein Vergleichsbeispiel und nicht erfindungsgemäß.

### Sensorische Evaluierunq der Intensität

Die Intensität der erfindungsgemäßen Zusammensetzung P2 sowie der entsprechende Referenz Ref2 während eines Applikationscyclus wurde von einem sensorischen Panel beurteilt. Hierzu wurden einem Proband je 2 Sprühstöße aus einem Dispenser mit der erfindungsgemäßen Zusammensetzung P2 auf einen Unterarm appliziert sowie 2 Sprühstöße der Referenz Ref2 auf den anderen Unterarm.

Die Intensität des jeweiligen Parfüms (Zusammensetzung) wurde nach 1 h, 2 h, 3 h und 4 h von 16 trainierten Panelisten beurteilt.

Tabelle 1 zeigt die jeweilige Beurteilung der 16 Panelisten in Bezug auf die geruchliche Intensität der jeweiligen Zusammensetzung.

**Tabelle 1:**

| **Zeit** | **P2 stärker** | **Kein Unterschied** | **Ref2 stärker** |
|---|---|---|---|
| 1 h | 13/16 | 2/16 | 1/16 |
| 2 h | 14/16 | 2/16 | 0/16 |
| 3 h | 14/16 | 2/16 | 0/16 |
| 4 h | 13/16 | 3/16 | 0/16 |

### Beispiel F1: Deo-Pumpspray

| **Inhaltsstoffe** | **P1** **Gew.-%** |
|---|---|
| CaCl₂ | 4,0 |
| Riechstoffmischung aus Beispiel 1 | 0,8 |
| Parfümbase B1 | 0,4 |
| Ethanol | 74,1 |
| Wasser | 19,0 |
| Alpha-Tocopherol | 0,20 |
| Cocoamidopropyl PG-dimonium chloridphosphat | 0,40 |
| Chremophor RH 40 | 0,70 |
| Rosmarinextrakt, in Ethanol löslich | 0,20 |
| Myristylalkohol | 0,20 |
| Total: | 100 |

| | |
|---|---|
| Chremophor RH 40: Polyoxyethylenglycerol-tri-hydroxystearat 40, Poly(oxyethylen)-40-hydriertes Rizinusöl (BASF AG) | |

Die Parfümbase B1 bestand aus:
11 Gew.-% Benzylacetat, 5 Gew.-% Cyclohexylacetat, 3 Gew.-% Decanol, 4 Gew.-% Amylzimtaldehyd, 1 Gew.-% 3a,6,6,9a-Tetramethyldodecahydronaphtho[2,1-b]furan (Synambran (10%ig in DPG)), 9 Gew.-% Rosenholzöl, 4 Gew.-% Allylcaproat, 10 Gew.-% Geraniol, 4 Gew.-% 8-Cyclohexadecenon, 4 Gew.-% Eugenol, 10 Gew.-% Methyldihydrojasmonat (Hedion®), 12 Gew.-% Jasmin absolue, 4 Gew.-% Ylang Ylang Öl, 3 Gew.-% 2-Methyl-3-(4-tert.-butylphenyl) propanal (Lilial®), 5 Gew.-% 2-Methyl-5-phenylpentanol (Rosaphen), 4 Gew.-% 2-Heptylcyclopentanon, 3 Gew.-% Ethylenbrassylat, 2 Gew.-% Dihydrocumarin, 1 Gew.-% Vanillin.

### Beispiel F2: Aerosol-Pumpspray

| **Inhaltsstoffe** | **P1** **Gew.-%** |
|---|---|
| CaCl₂ | 4,48 |
| Riechstoffmischung aus Beispiel 1 | 1,1 |
| Parfümbase B1 aus Beispiel F1 | 0,8 |
| Ethanol | 87,0 |
| Wasser | 5,0 |
| Alpha-Tocopherol | 0,20 |
| Hydroxypropylcellulose | 0,20 |
| Rosmarinextrakt, in Ethanol löslich | 0,22 |
| Cetylalkohol | 1,00 |
| Total: | 100 |

Die angegebenen Inhaltsstoffe wurden homogenisiert und anschließend 60 Gewichtsteile dieser Mischung mit 40 Gewichtsteilen Dimethylether (Treibmittel) in einer Spraydose abgefüllt.

### Beispiel F3: Nonaerosol-Haarspray mit extra starker Festigung

| | I(Gew.-%) | II (Gew.-%) |
|---|---|---|
| Ethanol | 58,00 | 58,00 |
| Riechstoffmischung aus Beispiel 1 | 0,30 | 0,30 |
| Calciumchlorid (CaCl₂) | 2,00 | 2,00 |
| Zinkchlorid (ZnCl₂) | 1,00 | - |
| Lithiumchlorid (LiCl) | 1,00 | 3,00 |
| Sulfonierte Polyester (z.B. Kammpolymere gemäß DE 199 09 757) | 8,00 | 10,00 |
| Vitamin-E-acetat und Konservierungsmittel | 0,10 | 0,10 |
| UV-Filter | 0,10 | 0,10 |
| Wasser dest. | Ad 100,00 | Ad 100,00 |

## Patentansprüche

1. Zusammensetzung umfassend oder bestehend aus:
A) 45 - 99 Gew.-% Ethanol, bezogen auf das Gesamtgewicht der Zusammensetzung,
B) einem oder mehreren Riechstoffen,
C) Wasser,
D) (i) einem oder mehreren deliqueszenten Stoffen.

2. Zusammensetzung nach Anspruch 1, wobei der Anteil der Summe der Bestandteile A), B), C) und D) an der Zusammensetzung 95 Gew.-% oder mehr, bevorzugt 98 Gew.-% oder mehr beträgt.

3. Zusammensetzung nach einem der vorangehenden Ansprüche, wobei in Bestandteil D) (i) der eine oder die mehreren deliqueszenten Stoffe ein bzw. mehrere Salze sind.

4. Zusammensetzung nach einem der vorangehenden Ansprüche, wobei Bestandteil D) (i) ein oder mehrere deliqueszente Salze umfasst, die ausgewählt sind aus einer Gruppe G
wobei die Gruppe G eine Gruppe ist bestehend aus:
Ammoniumhydrogencarbonat, Ammoniumtricitrat, Ammoniumfluorid, Ammoniumformiat, Ammoniummagnesiumchlorid, Ammoniumpropionat, Ammoniumsulfamat, Ammoniumthiocyanat, Bariumiodid, Bariumbromid, Bariumchlorid, Bariumthiocyanat, Bariumnitrat, Cäsiumiodid, Cäsiumchlorid, Cäsiumhydroxid, Cäsiumcarbonat, Cäsiumfluorid, Calciumbromid, Calciumchlorid, Calciumiodid, Calciumnitrat, Calciumnitrit, Calciumthiocyanat, Cobaltdiacetat, Cobaltdibromid, Cobaltdichlorat, Cobaltdiperchlorat, Cobaltdiiodid, Kupferdibromid, Kupferdichlorat, Kupferdiperchlorat, Kupferfluorosilicat, Kupferdinitrat, Lithiumchlorat, Lithiumchlorid, Lithiumbromid, Lithiumsalicylat, Lithiumsulfid, Magnesiumacetat, Magnesiumbromid, Magnesiumchlorat, Magnesiumperchlorat, Magnesiumchlorid, Magnesiumiodid, Magnesiumnitrat, Mangandichlorid, Kaliumiodid, Kaliumformiat, Kaliumhydroxid, Silberperchlorat, Natriumperchlorat, Natriumhydroxid, Natriumphenolat, Natriumhypophosphit, Natriumhydrogensulfid, Natriumsulfid, Natriumthiocarbonat, Natriumthiocyanat, Titantetrabromid, Titantrichlorid, Zinkperchlorat, Zinkchlorid und Zinkthiocyanat.

5. Zusammensetzung nach Anspruch 4, wobei Gruppe G eine Gruppe ist bestehend aus:
Calciumchlorid, Calciumnitrat, Kupferdinitrat, Lithiumchlorid und Magnesiumchlorid.

6. Zusammensetzung nach einem der vorangehenden Ansprüche, wobei Bestandteil D) in der Zusammensetzung vollständig gelöst vorliegt.

7. Zusammensetzung nach einem der vorangehenden Ansprüche, wobei Bestandteil D) in der Zusammensetzung in einer wirksamen Menge vorliegt, wobei eine wirksame Menge vorliegt, wenn in der Zusammensetzung durch den Bestandteil D) einer oder mehrere Effekte bewirkt werden, die ausgewählt sind aus der Gruppe von Effekten bestehend aus:
- Reduzierung der Verdampfung von Riechstoffen, insbesondere unmittelbar nach der Applikation und insbesondere von leichtflüchtigen Riechstoffen;
- zeitliche Stabilisierung des Geruchsprofils;
- Erhöhung oder zeitliche Stabilisierung des geruchlichen Impact (der wahrgenommenen Geruchsstärke);
- Verlängerung der Haftung der Zusammensetzung, insbesondere der leichtflüchtigen Riechstoffe (Kopfnote), auf Haut (d.h. nicht (Mund)Schleimhaut) und/oder Haar, insbesondere auf menschlicher Haut;
- Erhöhung oder Verlängerung der Diffusivität (Raumwirkung) der Zusammensetzung, insbesondere der leichtflüchtigen Riechstoffe (Kopfnote), auf Haut (d.h. nicht (Mund)Schleimhaut) und/oder Haar, insbesondere auf menschlicher Haut;
- Vermittlung eines angenehmen Hautgefühls.

8. Zusammensetzung nach einem der vorangehenden Ansprüche, umfassend:
A) 50 - 98,5 Gew.-%, vorzugsweise 60 - 95 Gew.-%, Ethanol,
und/oder
B) 0,1 - 35 Gew.-%, vorzugsweise 0,5 - 25 Gew.-%, einer Riechstoffmischung umfassend 2, 3, 4, 5, 6, 7, 8, 9, 10 oder mehr Riechstoffe,
und/oder
C) 0,5 - 40 Gew.-%, vorzugsweise 0,5 - 25 Gew.-%, Wasser,
und/oder
D) 0,5 - 20 Gew.-%, vorzugsweise 2 - 12 Gew.-%, bevorzugt 3 - 8 Gew.- %, eines oder mehrerer deliqueszenter Salze, sowie
sowie
E) gegebenenfalls Glycerin, Ethylenglykol, 1,2-Propylenglykol, Diethylphthalat und/oder ein geradkettiges Alkandiol mit 6 bis 12 C-Atomen,
wobei Gewichtsprozentangaben bezogen sind auf das Gesamtgewicht der Zusammensetzung.

9. Zusammensetzung nach einem der vorangehenden Ansprüche, umfassend oder bestehend aus:
A) 60 - 95 Gew.-% Ethanol,
B) 1-25 Gew.-% einer Riechstoffmischung umfassend 2, 3, 4, 5, 6, 7, 8, 9, 10 oder mehr Riechstoffe,
C) 1 - 22 Gew.-% Wasser,
D) 2 - 12 Gew.-%, bevorzugt 3 - 8 Gew.-%, eines oder mehrerer deliqueszenter Salze, sowie
E) gegebenenfalls Glycerin, Ethylenglykol, 1,2-Propylenglykol, Diethylphthalat und/oder ein geradkettiges Alkandiol mit 6 bis 12 C-Atomen,
wobei Gewichtsprozentangaben bezogen sind auf das Gesamtgewicht der Zusammensetzung.

10. Zusammensetzung nach einem der vorangehenden Ansprüche, wobei die Zusammensetzung eine, zwei oder sämtliche Eigenschaften aus der Gruppe besitzt, die besteht aus: transparent (klar), echte Lösung, sprühfähig (sprühbar).

11. Zusammensetzung nach einem der vorangehenden Ansprüche, wobei Bestandteil B) der Zusammensetzung einen oder mehrere Riechstoffe umfasst aus der Gruppe bestehend aus:
alpha-Pinen, beta-Pinen, Limonen, 6-Methyl-5-hepten-2-on, Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al, 3-Hexenylmethylcarbonat, Benzaldehyd, Linalool, Linalylacetat, Caryophyllen, Ethyllinalool, Citral, Neral, Geranial, Benzylalkohol, p-Anisaldehyd, Menthol, Hexylacetat, Citronellol, Nerol, Geraniol und 2-Phenylethylalkohol.

12. Zusammensetzung nach einem der vorangehenden Ansprüche, wobei Bestandteil B) eine Riechstoffmischung mit 2, 3, 4, 5, 6, 7, 8, 9, 10 oder mehr Riechstoffen ist, umfassend oder bestehend aus
i) einer Kopfnote, enthaltend oder bestehend aus einem oder mehreren Riechstoffen mit einem jeweiligen Dampfdruck bei 25°C von größer oder gleich 1,333 Pa.

13. Zusammensetzung nach einem der vorangehenden Ansprüche, wobei Bestandteil B) eine Riechstoffmischung mit 2, 3, 4, 5, 6, 7, 8, 9, 10 oder mehr Riechstoffen ist, umfassend oder bestehend aus
i) einer Kopfnote, enthaltend oder bestehend aus einem oder mehreren Riechstoffen mit einem jeweiligen Dampfdruck bei 25°C von größer oder gleich 1,333 Pa, und
ii) einem oder mehreren Riechstoffen mit einem jeweiligen Dampfdruck bei 25°C von kleiner als 1,333 Pa.

14. Zusammensetzung nach einem der vorangehenden Ansprüche, wobei Bestandteil B) eine Riechstoffmischung mit 2, 3, 4, 5, 6, 7, 8, 9, 10 oder mehr Riechstoffen ist, umfassend oder bestehend aus
i) einer Kopfnote, enthaltend oder bestehend aus 2, 3, 4, 5, 6 oder mehr Riechstoffen mit einem jeweiligen Dampfdruck bei 25°C von größer oder gleich 1,333 Pa, und
ii) 2, 3, 4, 5, 6 oder mehr Riechstoffen mit einem jeweiligen Dampfdruck bei 25°C von kleiner als 1,333 Pa.

15. Zusammensetzung nach einem der vorangehenden Ansprüche, zusätzlich umfassend
F) eine oder mehrere Substanzen aus der Gruppe bestehend aus: Antioxidantien, BHA, Tocopherol, hautpflegende Stoffe, Citronensäure, Vitamin C, UV-Filter, Emulgatoren, Fettalkohole, C₈-C₃₀-Fettsäure-alkylester und Farbstoffe.

16. Zusammensetzung nach Anspruch 15, wobei Bestandteil F) in einer Menge von 2 Gew.-% oder weniger vorliegt, bezogen auf das Gesamtgewicht der Zusammensetzung.

17. Zusammensetzung nach einem der vorangehenden Ansprüche, wobei die Zusammensetzung ausgewählt ist aus der Gruppe bestehend aus: Extrait, Eau de Parfum, Eau de Toilette, Eau de Cologne, Splash Cologne, Rasierwasser, Deo-Formulierungen, Haarfestiger, Haarspray, Haargele, Gesichtswasser, Haarwasser, Handdesinfektionsmittel, Flüssigkeiten und Emulsionen für Erfrischungstücher.

18. Verwendung eines deliqueszenten Stoffes als Mittel zur
- Reduzierung der Verdampfung von Riechstoffen in einer ethanolischen Riechstoffzusammensetzung, insbesondere unmittelbar nach der Applikation und insbesondere von leichtflüchtigen Riechstoffen;
- zeitlichen Stabilisierung des Geruchsprofils einer ethanolischen Riechstoffzusammensetzung;
- Erhöhung oder zeitlichen Stabilisierung des geruchlichen Impact (der wahrgenommenen Geruchsstärke) einer ethanolischen Riechstoffzusammensetzung;
- Verlängerung der Haftung von Riechstoffen einer ethanolischen Riechstoffzusammensetzung, insbesondere der leichtflüchtigen Riechstoffe (Kopfnote), auf Haut und/oder Haar, insbesondere auf menschlicher Haut;
- Erhöhung oder Verlängerung der Diffusivität (Raumwirkung) einer ethanolischen Riechstoffzusammensetzung, insbesondere der leichtflüchtigen Riechstoffe (Kopfnote), auf Haut und/oder Haar, insbesondere auf menschlicher Haut;
und/oder
- Vermittlung oder Verstärkung eines angenehmen Hautgefühls einer ethanolischen Riechstoffzusammensetzung.

19. Verfahren zur
- Herstellung einer Zusammensetzung nach einem der Ansprüche 1 bis 17,
- Reduzierung der Verdampfung von Riechstoffen in einer ethanolischen Riechstoffzusammensetzung, insbesondere unmittelbar nach der Applikation und insbesondere von leichtflüchtigen Riechstoffen,
- zeitlichen Stabilisierung des Geruchsprofils einer ethanolischen Riechstoffzusammensetzung,
- Erhöhung oder zeitlichen Stabilisierung des geruchlichen Impact (der wahrgenommenen Geruchsstärke) einer ethanolischen Riechstoffzusammensetzung,
- Verlängerung der Haftung von Riechstoffen einer ethanolischen Riechstoffzusammensetzung, insbesondere der leichtflüchtigen Riechstoffe (Kopfnote), auf Haut (d.h. nicht (Mund)Schleimhaut) und/oder Haar, insbesondere auf menschlicher Haut,
- Erhöhung oder Verlängerung der Diffusivität (Raumwirkung) einer ethanolischen Riechstoffzusammensetzung, insbesondere der leichtflüchtigen Riechstoffe (Kopfnote), auf Haut (d.h. nicht (Mund)Schleimhaut) und/oder Haar, insbesondere auf menschlicher Haut
und/oder
- Vermittlung oder Verstärkung eines angenehmen Hautgefühls einer ethanolischen Riechstoffzusammensetzung,
mit folgendem Schritt:
Mischen von
A) 45 - 99 Gew.-% Ethanol,
B) einem oder mehreren Riechstoffen,
C) Wasser,
sowie gegebenenfalls weiteren Bestandteilen mit
D) (i) einem oder mehreren deliqueszenten Stoffen und/oder (ii) zwei oder mehr Verbindungen, die in der resultierenden Zusammensetzung zusammen in-situ einen deliqueszenten Stoff bilden,
wobei die Gewichtsprozentangabe bezogen ist auf das Gesamtgewicht der resultierenden Zusammensetzung nach dem Mischen.

20. Verfahren zur Vermittlung, Verstärkung oder Modifizierung eines Geruches auf der menschlichen Haut, mit folgendem Schritt:
- Applizieren einer Zusammensetzung nach einem der Ansprüche 1 bis 17 auf die menschliche Haut.
